# EUROPEAN PATENT SPECIFICATION

(11) **EP 4 611 638 B1**
(45) Date of publication and mention of the grant of the patent: **21.01.2026**
(21) Application number: 24791433.6
(22) Date of filing: 23.10.2024
(51) Int. Cl.: A61B 6/03, G16H 50/20, A61B 6/04, G06T 7/00, G06N 3/08, A61B 6/00, A61B 6/46, A61B 6/50

(54) **SYSTEM AND METHOD FOR COMPUTATION AND VISUALIZATION OF A POSITION INDICATOR OF A TARGET ANATOMY**
SYSTEM UND VERFAHREN ZUR BERECHNUNG UND VISUALISIERUNG EINES POSITIONSANZEIGERS EINER ZIELANATOMIE
SYSTÈME ET PROCÉDÉ DE CALCUL ET DE VISUALISATION D'UN INDICATEUR DE POSITION D'UNE ANATOMIE CIBLE

(30) Priority: 06.11.2023 EP 23207944
(43) Date of publication of application: 10.09.2025
(73) Proprietor: Koninklijke Philips N.V., 5656 AG Eindhoven (NL)
(72) Inventor: MAY, Jan Marek, 5656 AG Eindhoven (NL); KOEPNICK, Johannes, 5656 AG Eindhoven (NL); BRUECK, Heiner Matthias, 5656 AG Eindhoven (NL); LUNDT, Bernd, 5656 AG Eindhoven (NL)
(74) Representative: Philips Intellectual Property & Standards
(86) International application number: PCT/EP2024/079927
(87) International publication number: WO 2025/098777

(56) References cited:
- EP-B1- 3 157 435
- US-A1- 2018 247 427
- US-A1- 2019 318 497
- KRÖNKE SVEN ET AL: "CNN-based pose estimation for assessing quality of ankle-joint X-ray images", PROGRESS IN BIOMEDICAL OPTICS AND IMAGING, SPIE - INTERNATIONAL SOCIETY FOR OPTICAL ENGINEERING, BELLINGHAM, WA, US, vol. 12032, 4 April 2022 (2022-04-04), pages 120321A - 120321A, XP060156232, ISSN: 1605-7422, ISBN: 978-1-5106-0027-0, DOI: 10.1117/12.2611734
- KÖPNICK JOHANNES ET AL: "Estimation of the ankle-joint space visibility in x-ray images using convolutional neural networks", 20230403, vol. 12464, 3 April 2023 (2023-04-03), pages 1246409 - 1246409, XP060177170, ISSN: 1605-7422, ISBN: 978-1-5106-6033-5, DOI: 10.1117/12.2651757

## Description

### FIELD OF THE INVENTION

The present invention relates to the field of medical imaging. In particular, the invention relates to the field of visualization of a position indicator of a target anatomy during an X-ray imaging and/or fluoroscopy imaging, and to a method for generating a visual representation of a position indicator of a target anatomy.

### BACKGROUND OF THE INVENTION

During medical imaging a key element for obtaining clinical valuable medical images is the correct patient positioning. Depending on the projection, the correct patient positioning requires a lot of experience. The quality of the patient positioning is judged based on a medical image received from a target anatomy. In the daily clinical praxis, the patient is positioned based on the experience of the X-ray device/system user. The medical personnel usually judges the medical image on respective positions of bones or other anatomical elements indicating a position of the target anatomy. Especially for orthopedic images, this may result in retakes and accordingly in additional radiation for the patient because the target anatomy is not placed ideally.

Documents US20180247427A1, US 2019/318497A1 and "CNN-based pose estimation for assessing quality of ankle-joint X-ray images", Krönke Sven et al, disclose known methods/systems for patient positioning aid during medical imaging.

### SUMMARY OF THE INVENTION

There may therefore be a need for a system for allowing a user to position a target anatomy, wherein the position should correspond to a correct patient positioning for a good quality medical imaging.

An object of the invention is to provide a system that allows a user of a medical imaging device/system to position a target anatomy correctly, in particular a system which visualizes the actual position of the target anatomy to a user.

The object of the present invention is solved by the subject matter of the independent claims where further embodiments are incorporated in the dependent claims. It is understood that the following aspect of the invention equally applies to the method, the medical system (for example X-ray system), to the computer program element and to the computer readable medium. Accordingly, any feature, function, step and/or element described in the following with reference to one aspect of the present disclosure equally applies to any other aspect of the present disclosure.

According to a first aspect of the invention a system for visualization of a position indicator of a target anatomy is provided. The system comprises a processor configured to receive an image from an image device, the image comprising target anatomy information, and to process the received target anatomy information using a neural network for predicting at least one position indicator of a position of the target anatomy. The processor is further configured to generate a visual representation of the position indicator of the target anatomy based on the analysis of the neural network, wherein the visual representation is visualized on a display, wherein the visual representation indicates by the position indicator whether the target anatomy is in a suitable position for a medical imaging process.

In the context of the present invention, the term "visualization" may be understood to describe an optical illustration or presentation, of a position indicator. The visualization may be displayed on any device which is able to indicate, illustrate to a user in a visual or optical form the position indicator of any target anatomy. For example, the visualization may be performed in an interface of a computer, or a medical imaging device itself.

In the context of the present invention, the term "position indicator" may be understood to describe a mark, characteristic, and/or sign which is configured to present, in particular visually, a position of a target anatomy to a user. In particular, the indicator is configured and designed such that a position relevant for taking a medical image is indicated.

In the context of the present invention, the term "target anatomy" may be understood to describe an anatomy which should be examined using a medical imaging device, for example such as an X-ray device, and/or fluoroscopy device. The target anatomy may be any part of a body of a patient which will be examined by the medical image device. In particular, the invention may focus on joint images where it is often very challenging to assess the correction action on how to improve the positioning, but the invention may not be limited to joint imaging.

In the context of the present invention, the term "optimal position" may be understood to describe a position which is suitable for a medical imaging process. When the optimal position is obtained, the target anatomy must not be repositioned, which reduces retakes of medical images and improves the quality of the taken medical image. Accordingly, with the correct position the quality of the medical image taken from the correctly positioned target anatomy is improved.

The invention, as described herein, allows to obtain clinical valuable medical images, for instance valuable X-ray radiographs. A key element is the correct positioning of the patient, in particular of a body part, the target anatomy, of the patient. The correct positioning may depend on the projection and requires a lot of experience by the radiographer. A retrospective positioning visualization and a prospective positioning visualization is proposed. Accordingly, the invention as described with the variety of embodiments herein enables a correct positioning of a target anatomy before taking (prospective) or after taking (retrospective) a medical image. Accordingly, a user may improve the patient positioning even before taking a medical image. Further, this visualization is easily understandable, because the position indicators and their ideal relative location are a well-known feature in the clinical practice for judging patient positioning.

According to the invention, the position indicator of the target anatomy is a position indicative set of bone contours. In particular, the position indicator comprises two or more bone contours of the target anatomy. The bone contours may commonly occur in pairs, hence it is advantageous to use at least two bone contours for judging the position quality of a target anatomy. The bone contours may be pairs of respective bone contours, which are well-known features in the clinical practice for judging positions of a target anatomy, such as a joint, or condyles. On the other hand, the position indicator may comprise more than two bone contours, hence the position indicator may not be limited to at least two bone contours, or to a pair of bone contours. The respective amount of the bone contours used for the position indicator may depend on the target anatomy to be examined.

According to an exemplary embodiment of the invention, the position indicative bone contour may be at least one contour of a condyle, or a contour of a joint space. For example, for a lateral knee projection it is important that the lateral and medial condyles overlap well, while for an ankle anterior-posterior projection the medial and lateral joint space should be clearly visible. The bone contours may be for example a contour of the lateral/medial articulated surface of the talus for the tibia and/or a contour of condyles such as femur condyles and humerus condyles which may be often used in clinical practice to reposition a patient. Other examples of such bone contours are the contours of both sides of a joint space present in lateral and anterior posterior projections of the knee, ankle or elbow. The bone contours may occur in pairs, and from this pair of bone contours the position of the target anatomy can be derived. The list of the above mentioned contours is not limiting, other position indicators may also be used.

According to an exemplary embodiment of the invention, the visual representation may comprise the displaying of the position indicator of the target anatomy overlaid over a background image of the target anatomy, wherein the background image is at least a live representation of the target anatomy. Hence, with the overlay over the background image, the positioning indicator may be associated with the respective target anatomy, and the user can clearly derive from this overlaid visualization whether the position of the target anatomy is correct. The live representation may be received from the image device. The image device may be part of the system for visualization or may be an external part. At least the image device may be in communication with the system for visualization for providing image data of the image device to the system, in particular to the processor. Accordingly, the image device is configured to provide the image information with respect to the target anatomy and the background image. Differently speaking, from the information received from the image device the system, in particular the processor, is configured to derive information with respect to the target anatomy and/or the background image for using this information for the visualization of the position indicator. The live representation of the target anatomy may be any illustration, suitable to visually indicate the target anatomy to a user.

According to an exemplary embodiment of the invention, the background image may be at least one of a live image of the target anatomy, a live depth image of the target anatomy, or a live schematic image of the target anatomy. Depending on the used technology for the image device, the background image may be displayed/illustrated as one of the above mentioned images. In particular, the image device may be a multidimensional image device configured to generate at least one of a 2D, or 3D image. For instance, a depth image may be generated using a depth image device configured to take a depth image, or a depth image map of the target anatomy. This image could be presented to the user via the visualization as background image. Another example may be the direct live image taken by a video camera as the image device, wherein a video of the target anatomy, for instance a video showing a knee of the patient, is used as the background image. A schematic image of the target anatomy may be an image wherein a silhouette of the target anatomy is illustrated, which schematically shows the (outer) shape of the target anatomy. When using a live image, the user may be able to move the target anatomy until the correct (ideal) position of the target anatomy is achieved such that a medical image having a good quality could be generated.

According to an exemplary embodiment of the invention, the neural network may be trained using data sets consisting of X-ray images of the target anatomy, depth images of the target anatomy, RGB images of the target anatomy. In the images used for training the neural network the relevant contours indicating a position of the target anatomy may be annotated, wherein the annotated position is at least one of a correct aligned position suitable for medical imaging and/or a misaligned position of the target anatomy. The neural network is trained with the herein mentioned information for being able to predict the relevant position indicator, for example the relevant pairs of bone contours. The neural network may be a trained deep neural network, wherein the invention may not be limited to this kind of neural network. The annotation of the relevant position indicators is carried out by persons with the necessary medical expertise. The annotated training data may comprise for example X-ray images and depth images or RGB images, wherein the bone contours are annotated in the X-ray images. This information is transferred to the depth and/or RGB images and the network is trained to predict the position of the contours based on the depth or RGB images. If a quality measure should be indicated by the system, the training data for the neural network may also comprise X-ray images, depth images and/or RGB images (one or more of each, or a combination thereof) in which the quality is annotated. Further, the trained neural network may be used in the system for visualization, to predict the position indicator, for instance the pairs of bone contours, on an image. The image may be a live stream of depth and/or RGB images.

According to an exemplary embodiment of the invention, the visual representation of the position indicator may comprise different line types for different positions. The different line types may be a continuous line, a dashed line, and/or a dotted line or any combinations thereof. Each position indicator may be indicated with a different line type, when the visualization for instance displays different position indicators. On the other hand, when the position indicator may comprise at least two bone contours, each bone contour may be indicated with a different line type to distinguish between these different bone contours in the visualization. For example, the line type may indicate the lateral and/or medial condyles. In the Figures the illustration of different line types with respect to the position indicators will be described in more detail.

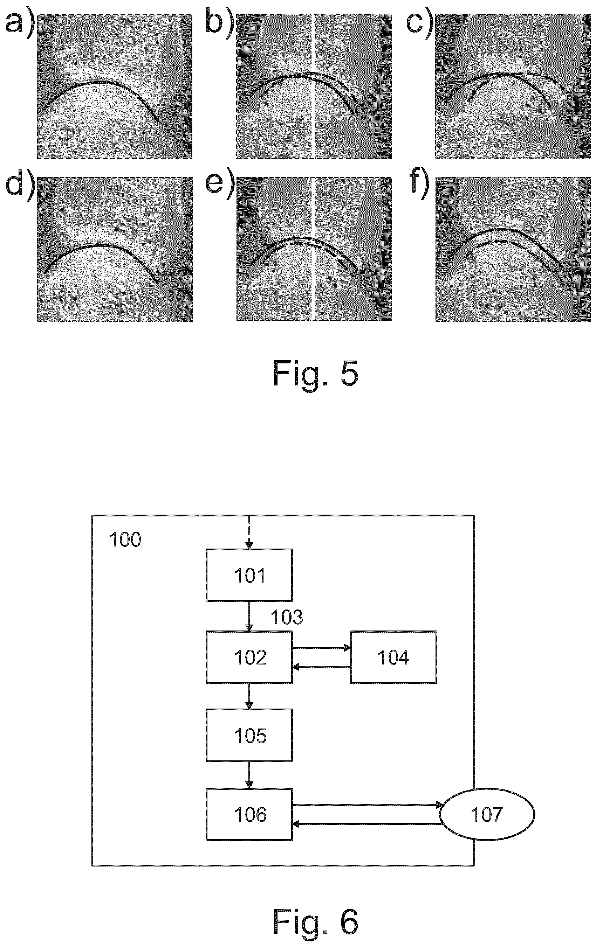

This visualization may also allow to assess the rotation direction how to improve the patient positioning by indicating for example the laterality of the bone contour. From the relative position of the bone contours, wherein each are indicated by a different line type, the user can derive how to correct the position of the target anatomy by rotation. More details are described with Figure 5.

According to an additional embodiment of the invention, a quality of the position may be indicated in the visual representation with different colors. For instance, for indicating a good quality, which means an ideal, correct position of the target anatomy the position indicators comprise the color green. On the other hand, when indicating a not-ideal quality orange may be chosen as color indicator, or for a bad quality a red color.

According to an exemplary embodiment of the invention, the visual representation may further comprise a guiding element configured for guiding the user to the correct position of the target anatomy suitable for a medical imaging process. For instance, the guiding element may be at least one arrow, or more arrows for indicating a direction of movement. The direction of movement indicated may be the direction into which the target anatomy may be moved for being aligned/positioned in the ideal position. Hence, the guiding element may be used to improve the visualization of a corrective action for the user.

According to an exemplary embodiment of the invention, when the position indicator may show that the target anatomy is not in the optimal position, the processor may be further configured to indicate to a user by the guiding element how to align the target anatomy. The processor may be configured to compare the imaged position with the ideal position of the target anatomy and based on this comparison result may calculate a correction movement for reaching the ideal position.

According to an exemplary embodiment of the invention, when the position indicator shows that the target anatomy is not in the optimal position, the processor is further configured to indicate with the position indicator that a system geometry of the medical imaging system is not correctly adjusted.

The processor may be in communication with the respective medical imaging system, and the visualization may indicate using the position indicator whether the position of the target anatomy has to be adapted or whether the system geometry, for example the tube angulation of an X-ray device, is not correct. The tube angulation, which should be corrected, may be derived by the respective position indicator. For example, when the position indicator may be at least two bone contours and they are spaced apart from each other the user may derive that the tube angulation needs to be corrected.

According to an exemplary embodiment of the invention, the guiding element may be at least one of a visual guiding element displayed on the display, or an acoustic guiding element. The guiding element may be a visual guiding element presented with the visualization of the position indicator on the background image. Additionally or alternatively the guiding element may be an acoustic guiding element. Different guiding elements may be applicable which allow a guiding of the user.

According to an exemplary embodiment of the invention, the system may further comprise an interface configured to allow a user to adjust the displaying of the visual representation of the target anatomy. The interface may be part of the system for visualization or it may be an external part. For instance, the system for visualization may use a processor which is in communication with a computer, or computer network, wherein the interface is part of this computer, computer network. Further, the interface may be a part of the medical imaging device with which the system for visualization is in communication.

According to an exemplary embodiment of the invention, the system may be further configured to adapt the visual representation when an alignment/position of the target anatomy is changed, wherein the position indicator is adapted to the change of the alignment of the target anatomy. In particular, when the background image of the visualization is a live view or live image of the target anatomy the movement of the target anatomy can be directly displayed/illustrated. The system for visualization is configured to adapt the position indicator during the movement of the target anatomy depending on the live image/view received from the image device. For instance, the processor may use the trained neural network to adapt the position indicator to the live view/image of the target anatomy.

According to an exemplary embodiment of the invention, the background image may be at least one of a medical image of the target anatomy, a RGB image of the target anatomy, a depth image of the target anatomy, or a schematic image of the target anatomy, received prior to the generation of the visual representation, wherein the visual representation is overlaid over the background image of the target anatomy for indicating by the position indicator whether the imaged target anatomy is in the optimal position.

With the visualization of the medical image as a background image, the system may be able to retrospectively visualize the position of the target anatomy. The user may derive from the visualization whether the position is correct and may correct the position, which means repositioning of the target anatomy, for performing a retake of the medical image. For instance, a pair of bone contours may be shown on an X-ray image and the user may be guided for repositioning to performing a retake. In the retrospective case, the background image may be most probably the X-ray image or an RGB, depth or schematic image from the point in time of the exposure.

According to a second aspect of the present invention, a method for generating a visual representation of a position indicator of a target anatomy is provided. The method comprising the steps of: receiving target anatomy information from an image device, wherein the image device receives an image of the target anatomy, processing the received target anatomy information using a neural network for predicting at least one position indicator of the target anatomy, generating a visual representation of the position indicator of the target anatomy based on an analysis of the neural network, displaying the visual representation on a display, indicating by the position indicator whether the position of the target anatomy is suitable for a medical imaging process.

The method may be further configured to perform/carry out steps representing the functions as described with the embodiments of the system for visualization.

According to a third aspect of the present invention a computer program product is provided. A computer program product or a computer program element is provided that is characterized by being adapted to execute the method steps of the method according to one of the preceding embodiments, on an appropriate system. The computer program product may comprise instructions, which, when the program is executed by a computer, cause the computer to carry out the method of any of the embodiments as described herein above. The computer program element might therefore be stored on a computer unit, which might also be part of an embodiment of the present invention. This computing unit may be adapted to perform or induce a performing of the steps of the method described above. The computing unit can be adapted to operate automatically and/or to execute the orders of a user. A computer program may be loaded into a working memory of a data processor. The data processor may thus be equipped to carry out the method of the invention. This exemplary embodiment of the invention covers both, a computer program that right from the beginning uses the invention and a computer program that by means of an update turns an existing program into a program that uses the invention. Further on, the computer program element might be able to provide all necessary steps to fulfill the procedure of an exemplary embodiment of the method as described above.

According to a further aspect of the invention a medical imaging system is provided, wherein the medical imaging system comprises the system for visualization according to any of the embodiments as described herein. The medical imaging system may be an X-ray system and/or a Fluoroscopy system. A medical imaging system equipped with the system as described with the various embodiments herein is capable of indicating, or visualizing to a user the correct position of the target anatomy and may prevent a retake due to non-correct position of the target anatomy. Further the image quality is increased due to the correct position of the target anatomy.

According to a further aspect of the present invention, a computer readable medium, such as a CD-ROM, is presented wherein the computer readable medium has a computer program element stored on it which computer program element is described by the preceding section. A computer program may be stored and/ or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. The computer-readable medium may comprise instructions which, when executed by a computer, cause the computer to carry out the method of any one of the embodiments as described herein.

It has to be noted that embodiments of the invention are described with reference to different subject matters. In particular, some embodiments are described with reference to method type claims whereas other embodiments are described with reference to the system/device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

### BRIEF DESCRIPTION OF THE DRAWINGS

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.
Fig 1 illustrates a visualization of a position indicator according to an embodiment of the invention.
Fig. 2 illustrates another visualization of a position indicator according to an embodiment of the invention.
Fig. 3 illustrates another visualization of a position indicator according to an embodiment of the invention.
Fig. 4 illustrates further visualizations of a position indicator according to an embodiment of the invention.
Fig. 5 illustrates a comparison of same kind of visualization of different positions according to an embodiment of the invention.
Fig. 6 illustrates a flow diagram illustrating different steps carried out by a system for visualization of a position indicator according to an embodiment of the invention.

### DETAILED DESCRIPTION OF EMBODIMENTS

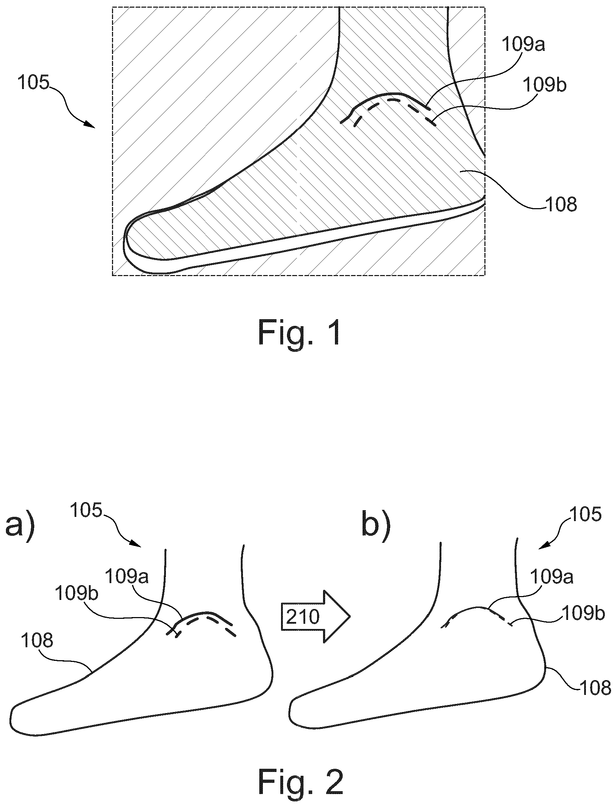

Fig 1 illustrates a visualization 105 of a position indicator 109 according to an embodiment of the invention. The visualization 105 may be displayed on a display or on an interface (not illustrated). In Fig.1 the visualization comprises a background image 108, which is a depth image of the target anatomy, wherein the different depths of the image (wherein a real depth information is a continuous information not a discrete as illustrated here in Fig. 1) are schematically illustrated with different hatchings. The background image may also be a live view/image, for example a live depth image, live schematic image, a live RGB image of the target anatomy. Illustrated are at least two position indicators 109 a and 109 b, which indicate a bone contour pair highlighting the displacement between the upper lateral and medial talus contours. Different line types are used for differentiating between the different contours belonging to the position indicator. In this case, the line type are used to distinguish between the upper lateral and medial talus contours. The dashed line position indicator 109b illustrates the lateral articulated surface of the talus contour and the continuous line position indicator 109a illustrates the medial articulated surface of the talus contour. As the lines do not overlap, the user can conclude from the visualization that the position of the target anatomy is not set properly and needs a correction. Accordingly, the user of the system for visualization can easily observe before taking the medical image that the patient positioning is not ideal. The user may now reposition the patient until the condyles overlap sufficiently, which is achieved if almost a single condyle contour is visible. For instance the positioning quality may be indicated to the user by switching from a red or orange color, if positioned wrong, to a green color, if positioned correctly.

Fig. 2 illustrates another visualization 105 of a position indicator according to an embodiment of the invention. In both Fig. 2a and Fig. 2b the target anatomy is an ankle of a foot. The visualization 105 comprises at least two position indicators 109 a, b indicating the bone contours of talus condyles, similar to the position indicators shown in Fig. 1. The background image 108 differs from the background image of Fig. 1. In Fig. 2 the background image 108 is a schematic of the foot, showing the outline of the foot. In Fig. 2 the position indicators 109 visualizes a not correct positioned feet, as the position indicators 109 a and b do not overlap, i.e. do not lay over each other for forming a single bone contour. In comparison thereto, the correct, ideal, position of the target anatomy is illustrated in the visualization of Fig. 2b. The correct position as illustrated in Fig. 2b may be achieved after a reposition performed by a user. Further, Fig. 2 illustrates a guiding element 210, which is an arrow, which indicates visually to a user that the target anatomy has to be repositioned. For instance, the arrow 210 may indicate the direction into which the foot has to be rotated for a correct position, in the case shown in Fig. 2 the arrow has to point upward or downward.

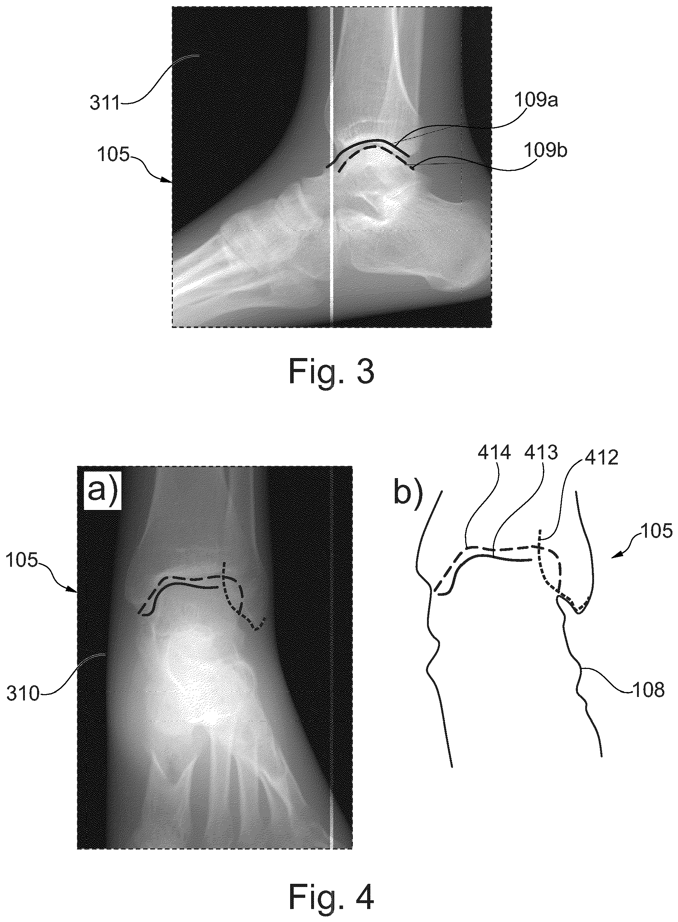

Fig. 3 illustrates another visualization 105 of a position indicator 109 according to an embodiment of the invention. The visualization comprises an X-ray image 311 and at least two position indicators 109a, b. The position indicator 109 comprises two bone contours of the lateral/medial articulated surface of the talus for the tibia 109a, b. Further, Fig. 3 illustrates a retrospective visualization 105 of a bone contour pair 109a, b highlighting the displacement between the talus condyles which is highly indicative for the quality of the patient positioning. As a retrospective visualization 105 the X-ray image is received before the position indicator 109 is calculated by the processor using the neural network. In this example a color may be used to indicate the quality while the line type visualizes the lateral 109b and medial condyles 109a.

Fig. 4 illustrates further visualizations 105 of a position indicator 109 according to an embodiment of the invention. The target anatomy in Fig. 4 is an anterior posterior ankle projection. As position indicator 109 pose indicative bone contours surrounding the ankle joint space are used. On the left side, Fig. 4 a), a retrospective visualization of the bone contours is shown. This means the background image 108 is an X-ray image of the target anatomy, the ankle. The right side, Fig. 4 b), shows a prospective visualization 105 based on a schematic image as background image 108 of the ankle contours and the live overlay of the predicted pose indicative contours 414, 412, 413. The 412 illustrates a fibula contour, the 413 illustrates a posterior contour of the articulated surface of the talus for the tibia, and the 414 illustrates an anterior contour of the articulated surface of the talus for the tibia. Hence as shown in Fig. 4 also more than two bone contours may be used to display the position of the target anatomy, such that the position indicator may comprise a plurality of bone contours.

Fig. 5 illustrates a comparison of visualizations 105 of position indicators for different positions of a target anatomy according to an embodiment of the invention. The two contours shown are part of one position indicator. In all Fig. 5 a) to f) the dashed line type indicates the contour of the lateral articulated surface of the talus for the tibia and the continuous line indicates the contour of the medial articulated surface of the talus for the tibia. Based on the displayed contours the user can correct the position by an appropriate repositioning. In practice, the positioning would be improved by leg rotation (5b and 5c) or by changing the tube angulation (5e and 5f). Fig. 5 a) and d) illustrate a perfect position, wherein no position error exists. In particular, a shift of the position indicators, which in Fig. 5 is indicated by the continuous line and the dashed line, the contour of the condyles illustrates the rotation, the movement of the target anatomy and the change, hence the adaption, of the position indicator to the movement. Fig 5 a) to c) illustrate a shift of the contours due to leg rotation. This means if the leg is rotated the bone contours may cross each other. On the other hand, when the bone contours are spaced apart from each other, hence are not crossing each other, a correction of the tube angulation may be necessary. Fig. 5 d) to f) illustrate a shift of the contours due to tube angulation. Hence, a change of the tube angulation may result therein that the bone contours diverge from each other. Also a combination of the correction of the position of the target anatomy by rotation and by the correction of the tube angulation may be possible.

Fig. 6 illustrates a flow diagram illustrating different steps carried out by a system 100 for visualization of a position indicator 109 according to an embodiment of the invention. The system 100 for visualization of a position indicator 109 of a target anatomy comprises a processor 102 configured to receive an image 103, or image information, from an image device 101, the image comprising target anatomy information, to process the received target anatomy information using a neural network 104 for predicting at least one position indicator 109 of a position of the target anatomy. The processor is further configured to generate a visual representation 105 of the position indicator 109 of the target anatomy based on the analysis of the neural network 104, wherein the visual representation 105 is visualized on a display 106. The visual representation 105 indicates by the position indicator 109 whether the target anatomy is in an optimal position for a medical imaging process. A user 107 may interact with the system 100. For instance, a guiding element generated from the system 100, in particular from the processor 102, may guide the user 107 to the correct position of the target anatomy suitable for a medical imaging process. The display 106, or any other interface, is configured to allow the user 107 to adjust the displaying of the visual representation on the display. Non limiting examples for the adjustment by the user are adjusting colors for different quality levels, adjusting limits to separate the different quality levels, adjusting the line type and thickness of lines, adjusting the background image type (RGB, depth, or schematic). The system described with Fig. 6 is able to carry out a method for generating a visual representation 105 of a position indicator of a target anatomy. The method comprising the steps of: receiving target anatomy information 103 from an image device 101, wherein the image device 101 generates an image of the target anatomy. The step of generating the image of the target anatomy may be understood, to describe that the image device may receive the image from a (RGB, depth) camera and/or and generates the image from the respective camera. On the other hand the image device itself may be the camera, wherein additional image processing components may be added. The method further comprises the steps of processing 102 the received target anatomy information using a neural network 104 for predicting at least one position indicator 109 of the target anatomy, generating a visual representation 105 of the position indicator 109 of the target anatomy based on an analysis of the neural network 104, displaying the visual representation 105 on a display 106, and indicating by the position indicator 109 whether the position of the target anatomy is suitable for a medical imaging process.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: system
- 101: image device
- 102: processor
- 103: image data
- 104: neural network
- 105: visualization
- 106: display
- 107: user
- 108: background image
- 109 a, b: position indicator
- 210: guiding element
- 310, 311: X-ray image
- 412: fibula contour
- 413: posterior contour of the articulated surface of the talus for the tibia
- 414: anterior contour of the articulated surface of the talus for the tibia

## Claims

1. A system (100) for visualization of a position indicator of a target anatomy, comprising
a processor (102) configured to receive an image (103) from an image device, the image comprising target anatomy information,
process the received target anatomy information using a neural network (104) for predicting at least one position indicator (109) of a position of the target anatomy,
generate a visual representation (105) of the position indicator of the target anatomy based on the analysis of the neural network, wherein the visual representation is visualized on a display (106),
wherein the visual representation indicates by the position indicator whether the target anatomy is in an optimal position suitable for a medical imaging process
**characterized in that**
the position indicator of the target anatomy is a position indicative set of bone contours (109a, 109b), in particular the position indicator comprises two or more bone contours of the target anatomy.

2. The system according to claim 1,
wherein the position indicative bone contour is at least one of a contour of condyle, or a contour of a joint space.

3. The system according to any of the preceding claims,
wherein the visual representation comprises the displaying of the position indicator of the target anatomy overlaid over a background image of the target anatomy,
wherein the background image is at least a live representation of the target anatomy.

4. The system according to claim 3,
wherein the background image is at least one of a live image of the target anatomy, a live depth image of the target anatomy, or a live schematic image of the target anatomy.

5. The system according to any of the preceding claims,
wherein the neural network is trained using data sets consisting of X-ray images of the target anatomy, depth images of the target anatomy, and/or RGB images of the target anatomy,
wherein in the images used for training the neural network the relevant bone contours indicating a position of the target anatomy are annotated,
wherein the annotated position is at least one of a correct aligned position suitable for medical imaging and/or a misaligned position of the target anatomy.

6. The system according to any of the preceding claims 1 to 5,
wherein the visual representation of the position indicator comprises different line types for different bone contours,
wherein a quality of the position is indicated in the visual representation with different colors.

7. The system according to any of the preceding claims,
wherein the visual representation further comprises a guiding element configured for guiding the user to the correct position of the target anatomy suitable for a medical image process.

8. The system according to any of the preceding claims,
wherein when the position indicator shows that the target anatomy is not in the optimal position, the processor is further configured to indicate with the position indicator that a system geometry of the medical imaging system is not correctly adjusted.

9. The system according to claim 7,
wherein the guiding element is at least one of a visual guiding element displayed on the display, or an acoustic guiding element.

10. The system according to any of the preceding claims, the system further comprising
an interface configured to allow a user to adjust the displaying of the visual representation of the target anatomy.

11. The system according to any of the preceding claims,
wherein the system is further configured to adapt the visual representation when an alignment of target anatomy is changed,
wherein the position indicator is adapted to the change of the alignment of the target anatomy.

12. The system according to any of the preceding claims,
wherein the image received from an imaging device, is at least one of a medical image of the target anatomy, a RGB image of the target anatomy, a depth image of the target anatomy, or a schematic image of the target anatomy received prior to the generation of the visual representation,
wherein the visual representation is overlaid over the background image of the target anatomy for indicating by the position indicator whether the imaged target anatomy is in the optimal position.

13. A method for generating a visual representation of a position indicator of a target anatomy, the method comprising the steps of:
receiving target anatomy information from an image device, device (101), wherein the image device generates an image (103) of the target anatomy,
processing the received target anatomy information using a neural network (104) for predicting at least one position indicator (109) of the target anatomy,
generating a visual representation (105) of the position indicator of the target anatomy based on an analysis of the neural network,
displaying the visual representation on a display (106),
indicating by the position indicator whether the position of the target anatomy is suitable for a medical imaging process,
**characterized in that**
the position indicator of the target anatomy is a position indicative set of bone contours (109a, 109b), in particular the position indicator comprises two or more bone contours of the target anatomy.

14. Computer program product comprising instructions, which when the program product is executed by a computer to cause the computer to carry out the method according to claim 13.

## Patentansprüche

1. System (100) zur Visualisierung eines Positionsindikators einer Zielanatomie, umfassend
einen Prozessor (102), der konfiguriert ist, um ein Bild (103) von einer Bildvorrichtung zu empfangen, wobei das Bild Zielanatomieinformationen umfasst,
die empfangenen Zielanatomieinformationen unter Verwendung eines neuronalen Netzes (104) zu verarbeiten, um mindestens einen Positionsindikator (109) einer Position der Zielanatomie vorherzusagen,
eine visuelle Darstellung (105) des Positionsindikators der Zielanatomie basierend auf der Analyse des neuronalen Netzes zu erzeugen, wobei die visuelle Darstellung auf einer Anzeige (106) visualisiert wird,
wobei die visuelle Darstellung durch den Positionsindikator angibt, ob die Zielanatomie in einer optimalen Position ist, die sich für einen medizinischen Bildgebungsprozess eignet
**dadurch gekennzeichnet, dass**
der Positionsindikator der Zielanatomie ein positionsindikativer Satz von Knochenkonturen (109a, 109b) ist, wobei der Positionsindikator insbesondere zwei oder mehr Knochenkonturen der Zielanatomie umfasst.

2. System nach Anspruch 1,
wobei die positionsindikative Knochenkontur mindestens eine von einer Gelenkkopfkontur oder einer Kontur Gelenkspalts ist.

3. System nach einem der vorstehenden Ansprüche,
wobei die visuelle Darstellung die Anzeige des Positionsindikators der Zielanatomie umfasst, der über einem Hintergrundbild der Zielanatomie überlagert ist,
wobei das Hintergrundbild mindestens eine Live-Darstellung der Zielanatomie ist.

4. System nach Anspruch 3,
wobei das Hintergrundbild mindestens eines von einem Live-Bild der Zielanatomie, einem Live-Tiefenbild der Zielanatomie oder einem schematischen Live-Bild der Zielanatomie ist.

5. System nach einem der vorstehenden Ansprüche,
wobei das neuronale Netzwerk unter Verwendung von Datensätzen trainiert wird, die aus Röntgenbildern der Zielanatomie und Tiefenbildern der Zielanatomie und/oder RGB-Bildern der Zielanatomie bestehen,
wobei in den Bildern, die zum Trainieren des neuronalen Netzes verwendet werden, die relevanten Knochenkonturen, die eine Position der Zielanatomie angeben, markiert sind,
wobei die markierte Position mindestens eine von einer korrekt ausgerichteten Position, die sich zur medizinischen Bildgebung eignet und/oder eine falsch ausgerichtete Position der Zielanatomie ist.

6. System nach einem der vorstehenden Ansprüche 1 bis 5,
wobei die visuelle Darstellung des Positionsindikators verschiedene Linienarten für unterschiedliche Knochenkonturen umfasst,
wobei eine Qualität der Position in der visuellen Darstellung durch unterschiedliche Farben angegeben wird.

7. System nach einem der vorstehenden Ansprüche,
wobei die visuelle Darstellung weiter ein Führungselement umfasst, das zum Führen des Benutzers zur richtigen Position der Zielanatomie konfiguriert ist, die sich für ein medizinisches Bildgebungsverfahren eignet.

8. System nach einem der vorstehenden Ansprüche,
wobei wenn der Positionsindikator zeigt, dass sich die Zielanatomie nicht in der optimalen Position befindet, der Prozessor weiter konfiguriert ist, um mit dem Positionsindikator anzugeben, dass eine Systemgeometrie des medizinischen Bildgebungssystems nicht korrekt angepasst ist.

9. System nach Anspruch 7,
wobei das Führungselement mindestens eines von einem visuellen Führungselement, das auf der Anzeige angezeigt wird, oder einem akustischen Führungselement ist.

10. System nach einem der vorstehenden Ansprüche, wobei das System weiter umfasst
eine Schnittstelle, die es einem Benutzer erlaubt, das Anzeigen der visuellen Darstellung der Zielanatomie anzupassen.

11. System nach einem der vorstehenden Ansprüche,
wobei das System weiter konfiguriert ist, um die visuelle Darstellung anzupassen, wenn eine Ausrichtung der Zielanatomie geändert wird.
wobei der Positionsindikator angepasst ist, um die Ausrichtung der Zielanatomie zu ändern.

12. System nach einem der vorstehenden Ansprüche,
wobei das von einer Bildgebungsvorrichtung empfangene Bild mindestens eines von einem medizinischen Bild der Zielanatomie, einem RGB-Bild der Zielanatomie, einem Tiefenbild der Zielanatomie oder einem schematischen Bild der Zielanatomie ist, das vor dem Erzeugen der visuellen Darstellung empfangen worden ist,
wobei die visuelle Darstellung zum Angeben durch den Positionsindikator über das Hintergrundbild der Zielanatomie überlagert wird, ob sich die abgebildete Zielanatomie in der optimalen Position befindet.

13. Verfahren zum Erzeugen einer visuellen Darstellung eines Positionsindikators einer Zielanatomie, wobei das Verfahren die Schritte umfasst zum:
Empfangen von Zielanatomieinformationen von einer Bildvorrichtung, Vorrichtung (101), wobei die Bildvorrichtung ein Bild (103) der Zielanatomie erzeugt,
Verarbeiten der empfangenen Zielanatomieinformationen unter Verwendung eines neuronalen Netzes (104) zum Vorhersagen mindestens eines Positionsindikators (109) der Zielanatomie,
Erzeugen einer visuellen Darstellung (105) des Positionsindikators der Zielanatomie basierend auf einer Analyse des neuronalen Netzes,
Anzeigen der visuellen Darstellung auf einer Anzeige (106),
Angeben durch den Positionsindikator, ob die Zielanatomie in einer optimalen Position ist, die sich für einen medizinischen Bildgebungsprozess eignet,
**dadurch gekennzeichnet, dass**
der Positionsindikator der Zielanatomie ein positionsindikativer Satz von Knochenkonturen (109a, 109b) ist, wobei der Positionsindikator insbesondere zwei oder mehr Knochenkonturen der Zielanatomie umfasst.

14. Computerprogrammprodukt, das Anweisungen umfasst, die, wenn das Programmprodukt von einem Computer ausgeführt wird, den Computer veranlassen, das Verfahren gemäß Anspruch 13 durchzuführen.

## Revendications

1. Système (100) de visualisation d'un indicateur de position d'une anatomie cible, comprenant
un processeur (102) configuré pour recevoir une image (103) en provenance d'un dispositif d'image, l'image comprenant des informations d"anatomie cible,
traiter les informations d"anatomie cible reçues à l'aide d'un réseau neuronal (104) pour prédire au moins un indicateur de position (109) d'une position de l'anatomie cible,
générer une représentation visuelle (105) de l'indicateur de position de l'anatomie cible sur la base de l'analyse du réseau neuronal, dans lequel la représentation visuelle est visualisée sur un écran (106),
dans lequel la représentation visuelle indique, par l'indicateur de position, si l'anatomie cible se trouve dans une position optimale appropriée pour un processus d'imagerie médicale
**caractérisé en ce que**
l'indicateur de position de l'anatomie cible est un ensemble indicatif de position de contours osseux (109a, 109b), en particulier l'indicateur de position comprend deux, ou plus, contours osseux de l'anatomie cible.

2. Système selon la revendication 1,
dans lequel le contour osseux indicatif de position est au moins un d'un contour de condyle ou d'un contour d'un espace articulaire.

3. Système selon l'une quelconque des revendications précédentes,
dans lequel la représentation visuelle comprend l'affichage de l'indicateur de position de l'anatomie cible superposé sur une image de fond de l'anatomie cible,
dans lequel l'image de fond est au moins une représentation en direct de l'anatomie cible.

4. Système selon la revendication 3,
dans lequel l'image de fond est au moins une d'une image en direct de l'anatomie cible, d'une image de profondeur en direct de l'anatomie cible ou d'une image schématique en direct de l'anatomie cible.

5. Système selon l'une quelconque des revendications précédentes,
dans lequel le réseau neuronal est entraîné à l'aide d'ensembles de données composés d'images radiographiques de l'anatomie cible, d'images de profondeur de l'anatomie cible et/ou d'images RVB de l'anatomie cible,
dans lequel, dans les images utilisées pour entraîner le réseau neuronal, les contours osseux appropriés indiquant une position de l'anatomie cible sont annotés,
dans lequel la position annotée correspond au moins à une d'une position correctement alignée appropriée pour une imagerie médicale et/ou une position mal alignée de l'anatomie cible.

6. Système selon l'une quelconque des revendications précédentes 1 à 5,
dans lequel la représentation visuelle de l'indicateur de position comprend différents types de lignes pour différents contours osseux,
dans lequel une qualité de la position est indiquée dans la représentation visuelle par différentes couleurs.

7. Système selon l'une quelconque des revendications précédentes,
dans lequel la représentation visuelle comprend en outre un élément de guidage configuré pour guider l'utilisateur vers la position correcte de l'anatomie cible appropriée pour un processus d'imagerie médicale.

8. Système selon l'une quelconque des revendications précédentes,
dans lequel, lorsque l'indicateur de position montre que l'anatomie cible n'est pas dans la position optimale, le processeur est en outre configuré pour indiquer avec l'indicateur de position qu'une géométrie du système d'imagerie médicale n'est pas correctement ajustée.

9. Système selon la revendication 7,
dans lequel l'élément de guidage est au moins un d'un élément de guidage visuel affiché sur l'écran ou d'un élément de guidage acoustique.

10. Système selon l'une quelconque des revendications précédentes, le système comprenant en outre
une interface configurée pour permettre à un utilisateur d'ajuster l'affichage de la représentation visuelle de l'anatomie cible.

11. Système selon l'une quelconque des revendications précédentes,
dans lequel le système est en outre configuré pour adapter la représentation visuelle lorsqu'un alignement de l'anatomie cible est modifié,
dans lequel l'indicateur de position est adapté au changement d'alignement de l'anatomie cible.

12. Système selon l'une quelconque des revendications précédentes,
dans lequel l'image reçue d'un dispositif d'imagerie est au moins une d'une image médicale de l'anatomie cible, d'une image RVB de l'anatomie cible, d'une image de profondeur de l'anatomie cible ou d'une image schématique de l'anatomie cible reçue avant la génération de la représentation visuelle,
dans lequel la représentation visuelle est superposée sur l'image de fond de l'anatomie cible pour indiquer, par l'indicateur de position, si l'anatomie cible imagée est dans la position optimale.

13. Procédé de génération d'une représentation visuelle d'un indicateur de position d'une anatomie cible, le procédé comprenant les étapes consistant à :
recevoir des informations d'anatomie cible en provenance d'un dispositif d'imagerie (101), dans lequel le dispositif d'image génère une image (103) de l'anatomie cible,
traiter les informations d'anatomie cible reçues à l'aide d'un réseau neuronal (104) pour prédire au moins un indicateur de position (109) de l'anatomie cible,
générer une représentation visuelle (105) de l'indicateur de position de l'anatomie cible sur la base d'une analyse du réseau neuronal,
afficher la représentation visuelle sur un écran (106),
indiquer par l'indicateur de position si la position de l'anatomie cible est appropriée pour un processus d'imagerie médicale,
**caractérisé en ce que**
l'indicateur de position de l'anatomie cible est un ensemble indicatif de position de contours osseux (109a, 109b), en particulier l'indicateur de position comprend deux, ou plus, contours osseux de l'anatomie cible.

14. Produit de programme informatique comprenant des instructions qui, lorsque le produit de programme est exécuté par un ordinateur, amènent l'ordinateur à exécuter le procédé selon la revendication 13.
